Europäisches Patentamt

European Patent Office

Office européen des brevets

(11) Publication number: **0 544 942 A1**

## (12) EUROPEAN PATENT APPLICATION

(21) Application number: **91120974.0**

(22) Date of filing: **06.12.91**

(51) Int. Cl.5: **C12N 15/12**, C12N 9/12, A61K 37/02, C12P 21/08, G01N 33/68, C07K 7/00, A61K 37/64

(43) Date of publication of application:
**09.06.93 Bulletin 93/23**

(84) Designated Contracting States:
**AT BE CH DE DK ES FR GB GR IT LI LU NL SE**

(71) Applicant: **Max-Planck-Gesellschaft zur Förderung der Wissenschaften e.V.**
**Bunsenstrasse 10**
**W-3400 Göttingen(DE)**

(72) Inventor: **Mandelkow, Eva-Maria, Dr.**
**Baron-Voght-Strasse 212a**
**W-2000 Hamburg 52(DE)**
Inventor: **Mandelkow, Eckhard, Prof. Dr.**
**Baron-Voght-Strasse 212a**
**W-2000 Hamburg 52(DE)**
Inventor: **Lichtenberg-Kraag, Birgit, Dr.**
**Weddingstrasse 5**
**W-1000 Berlin 65(DE)**
Inventor: **Biernat, Jacek, Dr.**
**Zeughausstrasse 12**
**W-2000 Hamburg 11(DE)**
Inventor: **Drewes, Gerard, Dr.**
**Kl. Schaeferkamp 40**
**W-2000 Hamburg 36(DE)**
Inventor: **Steiner, Barbara, Dipl.-Biol.**
**Parkgrund 12**
**W-2000 Schenefeld(DE)**

(74) Representative: **Vossius & Partner**
**Siebertstrasse 4 P.O. Box 86 07 67**
**W-8000 München 80 (DE)**

(54) **Novel tools for the diagnosis and treatment of Alzheimer disease.**

(57) The invention relates to epitopes of the tau protein which are specifically occurring in a phosphorylated state in tau protein from Alzheimer paired helical filaments, to a protein kinase which is responsible for the phosphorylation of the amino acids of the tau protein giving rise to said epitopes, and to antibodies specific for said epitopes. The invention further relates to pharmaceutical compositions for the treatment of Alzheimer disease, to diagnostic compositions and methods for the detection of Alzheimer disease and to the use of said epitopes for the generation of antibodies specifically detecting Alzheimer tau protein.

The invention relates to epitopes of the tau protein which are specifically occurring in a phosphorylated state in tau protein from Alzheimer paired helical filaments, to a protein kinase which is responsible for the phosphorylation of the amino acids of the tau protein giving rise to said epitopes, and to antibodies specific for said epitopes. The invention further relates to pharmaceutical compositions for the treatment of Alzheimer disease, to diagnostic compositions and methods for the detection of Alzheimer disease and to the use of said epitopes for the generation of antibodies specifically detecting Alzheimer tau protein.

The brains of Alzheimer patients contain two characteristic types of protein deposits, the plagues and the tangles. These structures have been of peak importance in Alzheimer research during the last few years (for a recent review see Goedert et al., Current Opinion in Neurobiology 1 (1991), 441 to 447). A prominent component of the tangles are the paired helical filaments (PHFs). It seems now clear that the PHFs are largely made up of the microtubule-associated protein tau which is normally attached to the neuronal microtubule network and, furthermore, particularly enriched in the axons.

There are six isoforms of tau in human brain that arise from alternative splicing of a single gene. All these isoforms also occur in PHFs (Goedert et al., Neuron 3 (1989), 519-526). The main biochemical differences between normal and Alzheimer PHF tau protein known so far may be summarized as follows:

(1) PHF tau protein is, in contrast to normal tau protein, highly insoluble which makes a biochemical analysis difficult;

(2) PHF tau protein reacts with certain antibodies in a phosphorylation dependent manner, suggesting a special phosphorylation status (Grundke-Iqbal et al., Proc. Natl. Acad. Sci. USA 83 (1986), 4913-4917, Nukina et al., Proc. Natl. Acad. Sci. USA 84 (1987), 3415-3419);

(3) PHF tau protein has a lower electrophoretic mobility in SDS gels, suggesting a higher $M_r$ value which may be related to its phosphorylation pattern (Steiner et al., EMBO J. 9 (1990), 3539-3544);

(4) PHF tau protein forms paired helical filaments with a characteristic 78 nm crossover repeat (Crowther and Wischek, EMBO J. 4 (1985), 3661-3665).

It has been hypothesized that PHF tau protein has a lower affinity for microtubules compared to normal tau proteins since a similar effect has been found when normal tau is phosphorylated in vitro by some kinases (Lindvall and Cole, J. Biol. Chem. 259 (1984), 5301-5305). Lack or reduced binding to microtubuli might therefore be a result of abnormal phosphorylation of the tau protein. This abnormal state might lead to microtubule disassembly and interfere with vital neuronal processes, such as rapid axonal transport. The abnormally phosphorylated tau proteins might then aggregate into PHFs. As a consequence thereof the neurons would eventually die thus setting the stage for the generation of the Alzheimer disease.

To date, it is not known which protein kinases are responsible for the abnormal phosphorylation. Ishiguro et al. (Neuroscience Letters 128, (1991), 195-198) have isolated a kinase fraction from bovine brain extracts which contain a protein kinase recognizing the serine/threonine proline motif. This kinase phosphorylated residues Ser 144, Thr 147, Ser 177 and Ser 315 of the tau protein. These residues differed from the ones previously reported (Coleman and Anderson, J. Neurochem. 54 (1990), 1548-1555, Iqbal et al., Proc. Natl. Acad. Sci. USA 86 (1989), 5646-5650, and Lee et al. Science 251 (1991), 675-678) which in turn are different from each other. Therefore, it remains unclear which protein kinase and which target amino acid residue(s) are involved in the generation of Alzheimer disease, if at all.

It is, moreover, of utmost importance for the diagnosis of Alzheimer disease, in particular at an early stage of the disease process, to develop antibodies which are specifically directed to epitopes on the protein which are associated with the Alzheimer state. A monoclonal antibody, TAU1, has been isolated which is capable of distinguishing between phosphorylated and non-phosphorylated forms of the tau protein (see, e.g., Lee et al., ibid.). However, this antibody specifically recognizes dephosphorylated tau protein which is seemingly not associated with the Alzheimer state. Another antibody, Alz 50 (Ksiezak-Reding et al., J. Biol. Chem. 263 (1988), 7943-7947) reacts with PHFs as well as with tau protein. Sternberger et al., Proc. Natl. Acad. Sci. USA 82 (1985), 4774-4776, have isolated an antibody, SMI 34, which recognizes a phosphorylated epitope common to Alzheimer tau protein and neurofilament protein. Finally, Lee et al. (ibid.) made antibodies directed to a phosphorylated peptide comprising the KSPV motif in the C-terminal region of the tau protein. All these antibodies known in the art have the disadvantage that for none of them it is known whether they recognize an epitope which is uniquely characteristic for the Alzheimer disease state.

Thus, the technical problem underlying the present invention was to provide a phosporylated epitope characteristic for the Alzheimer tau protein, a kinase activity which specifically catalyzes this phosphorylation, pharmaceutical compositions comprising inhibitors to said kinase, antibodies for recognizing said epitopes, diagnostic compositions containing said epitopes, kinase and/or antibodies methods for the in vitro diagnosis of Alzheimer disease and methods for the in vitro conversion of normal tau protein into Alzheimer tau protein.

EP 0 544 942 A1

The solution to the above technical problem is achieved by providing the embodiments characterized in the claims.

Accordingly, the present invention relates to an epitope of the tau protein which is specifically occurring in a phosphorylated state in tau protein from Alzheimer paired helical filaments.

The term "phosphorylated state in tau proteins from Alzheimer paired helical filaments" refers to a state of the tau protein where tau shows an upward $M_r$ shift, has a reduced binding to microtubules and is phosphorylated at ser or thr followed by pro.

Note: Amino acids are denoted by the one-letter or three-letter code; see e.g. Lehninger, Biochemistry, 2nd edition, Worth Publishers, New York, 1975, page 72.

There may be one or more epitopes of the tau protein which specifically occur in a phosphorylated state in Alzheimer paired helical filaments. These epitopes may, moreover, be phosphorylated by a single or different enzymes displaying phosphorylating activity.

In a preferred embodiment of the present invention, said epitope is specifically phosphorylated by a protein kinase from mammalian brain having the following biochemical properties:

(a) it phosphorylates ser-pro and thr-pro motifs in tau protein;
(b) it has an $M_r$ of 42 kD;
(c) it is activated by ATP and has a $K_m$ of 1.5 mM;
(d) it is activated by tyrosine phosphorylation; and
(e) it is recognized by an anti-MAP kinase antibody.

The term "ser-pro and thr-pro motifs" as used herein refers to a phosphorylatable ser or thr residue followed by a pro residue.

The term "anti-MAP kinase antibody" refers to an antibody which specifically recognizes a mitogen activated kinase (MAP). This kinase probably belongs to a family of closely related enzymes which have been referred to in the art by different names, e.g. MAP2 (microtubule-associated protein 2, see e.g. de Miguel et al., DNA and Cell Biology 10 (1991), 505-514) kinase, MBP (myelin basic protein) kinase or ERK1 (for a review, see Hunter, Meth. Enzym. 200 (1991), 1-37). MAP kinase is similar with respect to its biochemical properties to functionally similar enzymes from a variety of sources (Hunter, ibid.).

In another preferred embodiment of the present invention said epitope includes the phosphorylatable serine residues 46, 199, 202, 235, 296, 404 and/or 422 and/or the phosphorylatable threonine residues 50, 69, 111, 175, 181, 205, 212, 217 and/or 231.

The numbering of the amino acids was done in line with the largest human tau isoform, htau 40, see Goedert et al. (1989 ibid.).

The epitope of the invention may comprise one or more of the residues enumerated above. Moreover, the epitope of the present invention may comprise only one or more phosporylated serine residues, one or more phosphorylated threonine residues or a combination thereof. The actual composition of the epitope may be determined by methods which are known in the art. It is also clear to the person skilled in the art that other amino acids of the protein may contribute to the epitope which is recognized by an anti-MAP specific antibody.

In a further preferred embodiment of the present invention, said epitope comprises the amino acid sequences

KESPLQ, YSSPGSP, PGSPGT, YSSPGSPGTPGS, PKSPSS, YKSPVVS, GDTSPRH, MVDSPQL, PLQTPTE, LKESPLQTPTED, AKSTPTA, IGDTPSL, PAKTPPA, APKTPPS, PAKTPPAPKTPPS, SPGTPGS, RSRTPSL, SLPTPPT, RSRTPSLPTPPT, VVRTPPK, VVRTPPKSPSSA.

Again, it is to be understood that not all of the amino acids of the peptide necessarily contribute to the specific site actually recognized by the antibody. It is also possible that sugar components (?) or different amino acids of the protein due to the folding of the protein may contribute to the epitope (?).

Another object of the present invention is to provide a protein kinase which is capable of specifically converting tau protein to Alzheimer tau protein by phosphorylation of the amino acid motif ser-pro or thr-pro. Preferably, said protein kinase belongs to the class of MAP kinases. These kinases can be used for various purposes, e.g. for the in vitro conversion of tau protein into Alzheimer tau protein. The Alzheimer tau protein thus obtainable may be used to study e.g. substances which are capable of inhibiting its formation or the formation of PHFs. Moreover, they may be used for the development of drugs capable of dissolving said PHFs or for converting Alzheimer tau protein into normal tau protein. It is also conceivable that a system based on the ability of the protein kinase of the invention to convert normal into Alzheimer tau protein will

3

EP 0 544 942 A1

provide a well defined in vitro system for Alzheimer disease.

In a preferred embodiment of the invention, said protein kinase has the following biochemical properties:

(a) it phosphorylates ser-pro and thr-pro motifs in tau protein;

(b) it has an $M_r$ of 42 kD;

(c) it is activated by ATP and has a $K_m$ of 1.5 mM;

(d) it is activated by tyrosine phosphorylation; and

(e) it is recognized by an anti-MAP kinase antibody.

The term "$M_r$" is defined as the relative molecular weight determined by SDS gel electrophoresis.

In still another preferred embodiment of the invention, said protein kinase is obtainable by carrying out the following steps:

(a) homogenizing porcine brain in 10 mM Tris-HCl, pH 7,2, 5 mM EGTA, 2 mM DTT and a cocktail of protease inhibitors (leupeptin, aprotinin, pepstatin A, $\alpha$2-macroglobulin, PMSF (phenyl methyl sulphonyl fluoride));

(b) centrifugating the homogenate at 100,000 x g for 30 minutes at 4°C;

(c) removing the supernatant after centrifugation;

(d) precipitating the crude protein by ammonium sulfate precipitation;

(e) desalting the crude preparation by gel filtration;

(f) activating the crude enzyme by incubation in activation buffer;

(g) further purifying the crude preparation by ion exchange chromatography; and

(h) identifying the enzyme by Western blotting.

The term "activation buffer" is defined as a buffer comprising 25 mM Tris, 2 mM EGTA, 2 mM DDT, 40 mM p-nitrophenylphosphate, 10 $\mu$M okadaic acid, 2 mM MgATP, and protease inhibitors.

With respect to the actual conditions used for obtaining said kinase, a person skilled in the art will be able to deviate from the protocol outlined above and still obtain the kinase of the invention. Such a deviation may, e.g., concern the composition of the protease inhibitor cocktail of step (a): It is conceivable to use different inhibitors under the proviso that the kinase activity is not diminished or destroyed.

In a most preferred embodiment the present invention relates to a protein kinase which specifically phosphorylates serine residues 46, 199, 202, 235, 396, 422 and threonine residues 50, 69, 111, 175, 181, 205, 212, 217, 231 of the tau protein.

In another preferred embodiment of the present invention, said kinase is a protein kinase from human brain, porcine brain, or another source.

Another object of the invention is to provide pharmaceutical compositions containing a specific inhibitor for the protein kinase of the invention, optionally in combination with a pharmaceutically acceptable carrier and/or diluent.

The term "specific inhibitor for the protein kinase" refers to substances which specifically inhibit the enzymatic action of the protein kinase of the present invention. Inhibitors to enzymes such as protein kinases and their mode of action are well known in the art. For example, such an inhibitor may bind to the catalytic domain of the enzyme thus rendering it incapable of converting its substrate.

Said pharmaceutical composition may be administered to a patient in need thereof by a route and in a dosage which is deemed appropriate by the physician familiar with the case.

Pharmaceutically acceptable carriers and/or diluents are well known in the art and may be formulated according to the route of administration or the special disease status of the patient.

In a preferred embodiment the present invention relates to a pharmaceutical composition for the treatment of Alzheimer disease.

Again, said pharmaceutical composition may be administered to a patient in need thereof by route and in a dosage which is deemed appropriate by the physician handling the case.

In another preferred embodiment of the present invention, said pharmaceutical composition contains as the specific inhibitor at least one oligo- or polypeptide comprising an epitope of the invention.

The term "oligo- or polypeptide comprising an epitope of the invention" refers to peptides which in their two- or three-dimensional structure reconstitute the epitope of the invention which is specifically recognized by an antibody directed thereto. Moreover, said oligo- or polypeptides may solely consist of the amino acids representing said epitope(s) or they may comprise additional amino acids. The construction of such oligo- or polypeptides is well known in the art.

Another object of the invention is an antibody which specifically recognizes an epitope of the invention. Said antibody may be a serum derived or a monoclonal antibody. The production of both monoclonal and polyclonal antibodies to a desired epitope is well known in the art (see, e.g. Harlow and Lane, Antibodies, A Laboratory Manual, Cold Spring Harbor Laboratory, Cold Spring Harbor, 1988). Furthermore, said antibody may be a natural or an antibody derived by genetic engineering, such as a chimeric antibody derived by

4

techniques which are well understood in the art. Moreover, said antibody also refers to a fragment of an antibody which has retained its capacity to bind the specific epitope, such as an Fab fragment.

In a preferred embodiment, the antibody of the present invention recognizes the protein kinase of the present invention.

The term "recognizes the protein kinase of the present invention" as used herein means that the antibody does not or insignificantly cross-reacts with other substances such as different protein kinases present in the same biological environment. Moreover, it means that the antibody does not or insignificantly cross-reacts with different protein kinases when tested in in vitro systems.

In another preferred embodiment, the antibody of the present invention is a monoclonal antibody.

Another object of the invention is to provide diagnostic compositions for the detection and/or monitoring of Alzheimer disease comprising

- an epitope of the invention;
- a kinase of the invention; and/or
- an antibody of the invention.

The diagnostic composition of the invention may comprise for example an antibody of the invention which specifically recognizes the kinase of the invention or an enhanced level of said kinase in a sample to be tested. In another embodiment, said diagnostic composition may comprise an antibody of the invention directed to the epitope of the invention. Thus, an Alzheimer correlated disease state of a sample may be detected by treating said sample with an antibody recognizing the epitope of the invention. The antibody-epitope (hapten) complex may be visualized using a second antibody directed to the antibody of the invention and being labelled according to methods known in the art (see, e.g., Harlow and Lane, ibid.).

In still another embodiment of the present invention, said diagnostic composition may consist of an epitope of the invention and an antibody of the invention. Treatment of a sample with said antibody may give rise to conclusions with regard to the disease state of the corresponding patent, if the binding of said antibody to said sample is brought in relation to binding of said antibody to said epitope of the invention used as a reference sample.

In still another embodiment, the diagnostic composition may comprise an epitope of the invention, a kinase of the invention and an antibody of the invention. Kinase activity may be monitored with respect to phosphorylation of the sample as compared to the phosphorylation of the epitope of the invention. From the quantitated kinase activity the phosporylation state of the tau protein contained in said sample and therefore the disease state of the patient may be deduced. The kinase activity may e.g. be deduced by including a substrate analog in the same reaction, which is visually detectable upon enzymatic conversion. Such substrate analogs are widely used in the art). Alternatively, the amount of phosphorylated tau protein in the sample may be detected after treatment with the kinase of the invention by employing an antibody of the invention directed to the phosphorylated epitope and using the amount of antibody-epitope complex provided by the diagnostic composition as an internal standard, or by determining the amount of phosphate incorporated into tau protein by the kinase, e.g. by radioactive tracer methods which are well known in the art.

The person skilled in the art is in the position to design other test systems which combine any of the above objects of the invention. It is to be understood that all conceivable combinations fall within the scope of protection of the present invention.

Another object of the invention is to provide a method for the in vitro diagnosis and/or monitoring of Alzheimer disease comprising assaying a cerebrospinal fluid isolate of a patient or carrying out a biopsy of nerve tissue

- for the presence of a phosphorylated Alzheimer tau protein containing an epitope of the invention; or
- for the presence of a protein kinase of the invention. The "cerebrospinal fluid isolate of a patient" is obtained by standard medical procedures.

An example for a nerve tissue suitable for said biopsy is the olfactory epithelium. The person skilled in the art may carry out said method employing e.g. the diagnostic tools illustrated in connection with the diagnostic compositions, supra.

In a preferred method of the present invention, the Alzheimer tau protein is detected by using an antibody of the invention.

Said antibody preferably is an antibody directed to the epitope of the invention.

In another preferred embodiment of the invention, the protein kinase is detected by using an oligo- or polypeptide comprising an epitope of the invention and/or by using an antibody of the invention.

Still another object of the invention is to provide a method for the in vitro conversion of normal tau protein into Alzheimer tau protein wherein normal tau protein is treated with a protein kinase of the present invention under conditions which allow the phosphorylation of said normal tau protein.

The term "Alzheimer tau protein" refers to tau protein that is abnormally phosphorylated (e.g. at ser-pro or thr-pro motifs) and recognized by Alzheimer-specific antibodies.

The term "conditions which allow the phosphorylation of said normal tau protein" refers to conditions allowing the activity, preferably the optimal activity, of protein kinase. This activity results in phosphorylation of the substrate at the ser-pro and/or thr-pro motifs. The phosphorylated substrate may then be recognized by Alzheimer-specific antibodies.

Normal tau protein may be derived from natural or recombinant sources. It is, for the purpose of carrying out the method of the present invention, however, expedient to use recombinant material.

The method of the present invention provides sufficient amounts of Alzheimer tau protein for a variety of purposes: With the method of the present invention an in vitro model for the study of the generation of the Alzheimer state of proteins may be established (see above). Moreover, inhibitors may be tested which prevent the conversion of normal to Alzheimer tau protein. These "inhibitors" may be specific for the epitope to be phosphorylated by e.g. blocking the epitope or may be directed to various domains on the protein kinase, as long as they prevent or disturb its biological activity. Furthermore, the Alzheimer tau protein generated by the method of the present invention may be employed in binding studies to microtubule structures thus contributing to the elucidation of the molecular basis underlying Alzheimer disease.

The person skilled in the art knows how to employ the method of the present invention for a variety of different purposes which all fall under the scope of protection of the present invention.

The present invention relates, moreover, to the use of an epitope of the invention for the generation of Alzheimer tau proteins specific antibodies.

The methods for obtaining said antibodies are well known in the art; thus, the generation of polyclonal or monoclonal antibodies may be conducted using standard methods (see, e.g., Harlow and Lane, ibid.). If an oligo- or polypeptide is used for the generation of antibodies it is desirable to couple the peptide comprising the epitope to a suitable carrier molecule capable of inducing or enhancing the immune response to said epitope, such as bovine serum albumin or keyhole limpet hemocyanin. The methods of coupling hapten (comprising or being identical to the epitope) and carrier are also well known in the art (Harlow and Lane, ibid.). It is also to be understood any animal suitable to generate the desired antibodies may be used therefor.

The Figures show:

Fig. 1:

> (a) SDS gel of tau isoforms, (b) immunoblots of (a) and PHF tau with the AT8 antibody.
> (a) SDS gel. Lane 1, marker proteins. Lane 2: Tau from bovine brain, showing several isoforms in a mixed state of phosphorylation. Lane 3, bovine brain tau after dephosphorylation with alkaline phosphatase. Note that all isoforms shift to a lower $M_r$. Lanes 4 and 5: Tau from normal human brain, before and after dephosphorylation. Lanes 6-11: bacterially expressed human tau isoforms htau23, 24, 37, 34, 39, 40 (see Goedert et al., 1989, ibid.). These isoforms have either three or four internal repeats of 31 residues each in the C-terminal half (three: htau23, 37, 39; four: htau24, 34, 40). Near the N-terminus there can be zero, one, or two inserts of 29 residues (zero: htau23, 24; one: htau37, 34; two: htau39, 40).
> (b) Immunoblots with the AT8 antibody. Lane 1, PHF tau, showing 4-6 isoforms in the range of 60-70 kD; all of them react strongly with AT8. Lanes 2-11, same preparations as in (a); none of the bovine or normal human tau isoforms show any reaction.

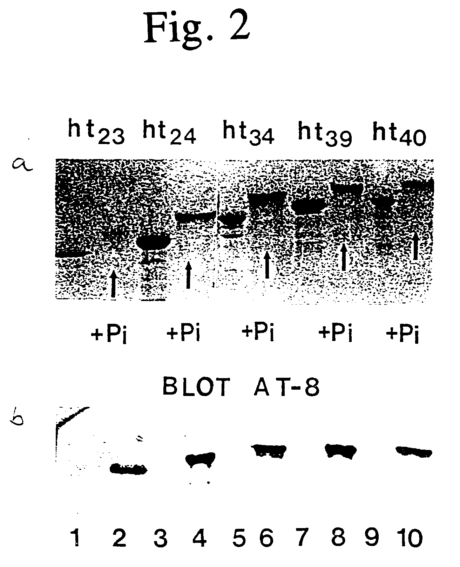

Fig. 2: Phosphorylation of bacterially expressed human tau isoforms with the kinase from brain. (a) SDS gels, (b) immunoblots with AT8.

> (a) Lanes 1 and 2, SDS gel of htau23 before and after extract phosphorylation (note the upward shift in $M_L$) Lanes 3-10 show analogous pairs for other isoforms (htau24, 34, 39, 40).
> (b) Immunoblots of (a) with AT8 antibody. It reacts with all tau isoforms after phosphorylation (even lanes; including htau37, not shown here).

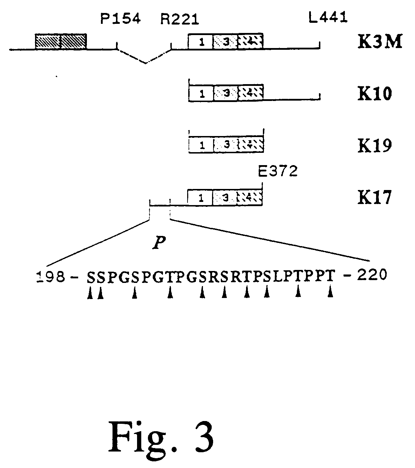

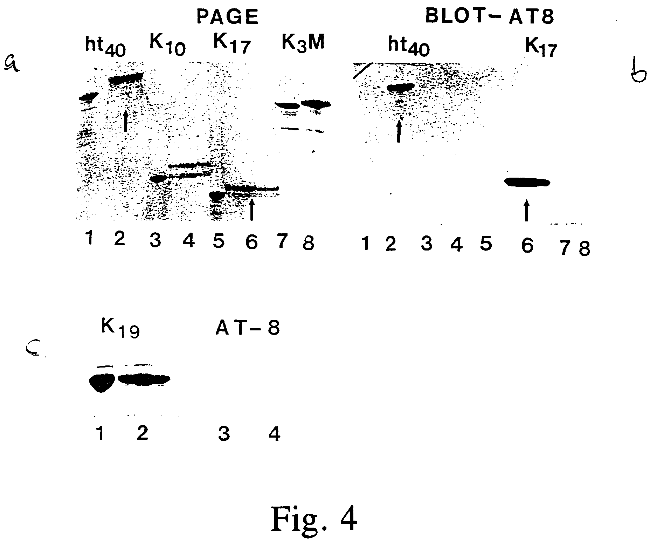

Fig. 3: Diagram of constructs K3M, K10, K19, and K17. K19 (99 residues) contains the sequence Q244-E372 of htau23 plus an N-terminal methionine. This comprises three of the repeats (repeat 1, 3, and 4; repeat 2 is absent in htau23). K10 (168 residues) is similar, except that it extends to the C-terminus of htau23 (L441). K17 (145 residues) contains the sequence S198-E372 (assembly domain starting at the chymotryptic cleavage site, up to end of fourth repeat, but without the second repeat, plus an N-terminal methionine). K3M (335 residues) contains the N-terminal 154 residues of bovine tau4, plus the sequence R221-L441 of htau23 (without second repeat). The location of peptide S198-T220 is indicated in K17. By comparison of the constructs the epitope of AT8 must be in this region (see Fig. 4).

Fig. 4: Phosphorylation of htau40 and constrcuts K10, K17, K3M, and K19.

(a) SDS gel. Odd lanes, htau40, K10, K17, and K3M before phosphorylation, even lanes, after phosphorylation. Note the upward shift of the bands after phosphorylation. In lane 4 there are two bands because K10 is not completely phosphorylated.

(b) Immunoblot of (a) with AT8. The antibody reacts only with htau40 (lane 2) and K17 (lane 6), both in the phosphorylated state, but not with K10 (lane 4) or K3M (lane 8), although these constructs are also phosphorylated and show an $M_r$ shift.

(c) Construct K19 before and after incubation with the kinase. Lanes 1 and 2, SDS gel; there is no $M_r$ shift and no phosphorylation, confirmed by autoradiography (not shown). Lanes 3 and 4, immunoblot with AT8, showing no reaction. This confirms that the epitope is not in the repeat region.

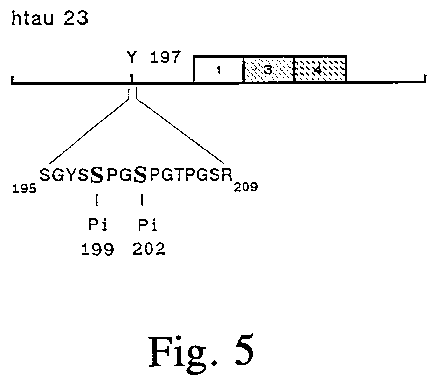

Fig. 5: Diagram of tryptic peptide S195-R209. The 15 residue peptide (containing 5 serines and 1 threonine) was labeled with two radioactive phosphates at S199 and S202, as determiend by sequencing.

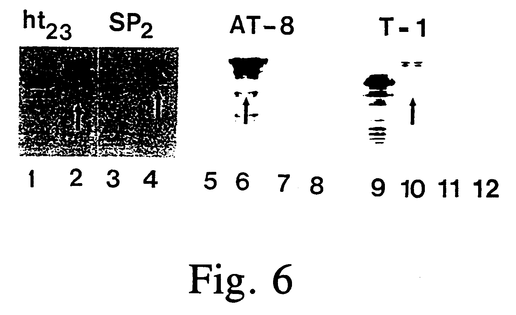

Fig. 6: Phosphorylation and antibody reactions of the D mutant of htau23 (S199 and S202 changed into D). Lanes 1 and 2, SDS gel of htau23 before and after extract phosphorylation; lanes 3 and 4, D-mutant before and after extract phosphorylation. Note that the D-mutant runs slightly higher than htau23 (lanes 1,3), but after phosphorylation both proteins have the same position in the gel (lanes 2, 4).

Lanes 5-8, immunoblots of lanes 1-4 with AT8. The antibody reacts only with extract phosphorylated htau23 (lane 6), but neither with the unphosphorylated form (lane 5) nor with the D-mutant (lanes 7, 8), although it was phosphorylated as seen by the additional shift and autoradiography (not shown).

Lanes 9-12, immunoblots of lanes 1-4 with TAU1. This antibody reacts only with htau23 before phosphorylation (lane 9), but not with the phosphorylated form (lane 10) nor with the D-mutant (lanes 11, 12). The aspartic acid apparently mimicks a phosphorylated serine and thus masks the epitope. The minor reaction of htau23 with TAU1 in lane 10 shows that the protein is not completely phosphorylated.

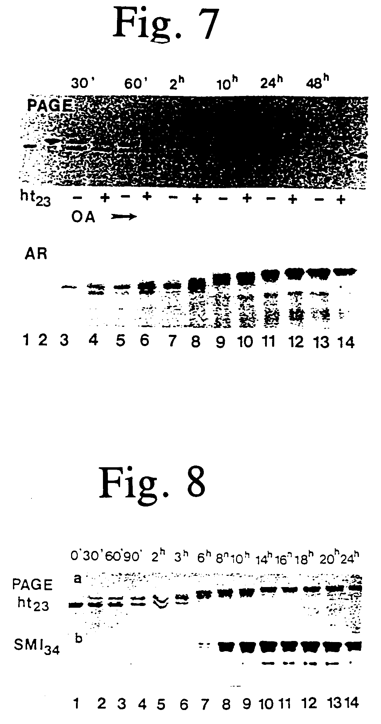

Fig. 7: Time course of phosphorylation of bacterially expressed human isoform htau23 with the brain kinase activity and corresponding autoradiogram.

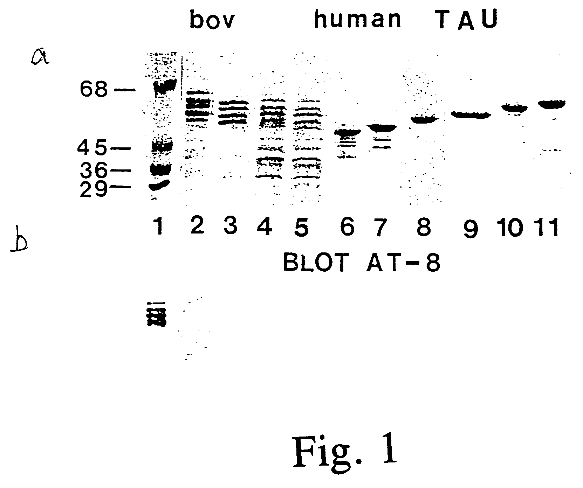

(a) SDS-PAGE of htau23 after incubation with the kinase between 0 and 24 hours, as indicated. The unphosphorylated protein is a single band of $M_{ro}$ = 48 kD (lane 1). Lanes 3-14 show that phosphorylation leads to a progressive shift to higher $M_r$ with well defined intermediate stages. The even lanes (numbered 4, 6, etc. below Fig. 1b) are observed in the presence of 10 $\mu$M okadaic acid (OA) (labeled " + " below Fig. 1a). The odd lanes (3, 5, etc. labeled "-") are without okadaic acid. The first stage takes about 2 hours (shift to a new $M_{r1}$ = 52 kD), the second is finished around 10 hours ($M_{r2}$ = 54 kD), the third is finished around time 24 hours ($M_{r3}$ = 56 kD); no further shift is observed during the subsequent 24 hours. Lane 2 shows a mutant that is not of significance in this context.

(b) Autoradiogram of (a). The quantitation of the phosphate incorporated (mol $P_i$/mol protein) in this experiment was as follows (-OA/ + OA): 30 min (0.5/1.0), 60 min (0.7/1.4), 120 min (1.0/2.0), 10 hours (2.0/3.0), 24 hours (3.2/4.0).

Fig. 8:

(a) SDS gel showing the time course of phosphorylation of htau23 similar to that of Fig. 1a, but with 10 $\mu$M okadaic acid throughout;

(b) immunoblot of (a) with the monoclonal antibody SMI34. The antibody recognizes the protein only in the second and third stage of phosphorylation, but not in the first.

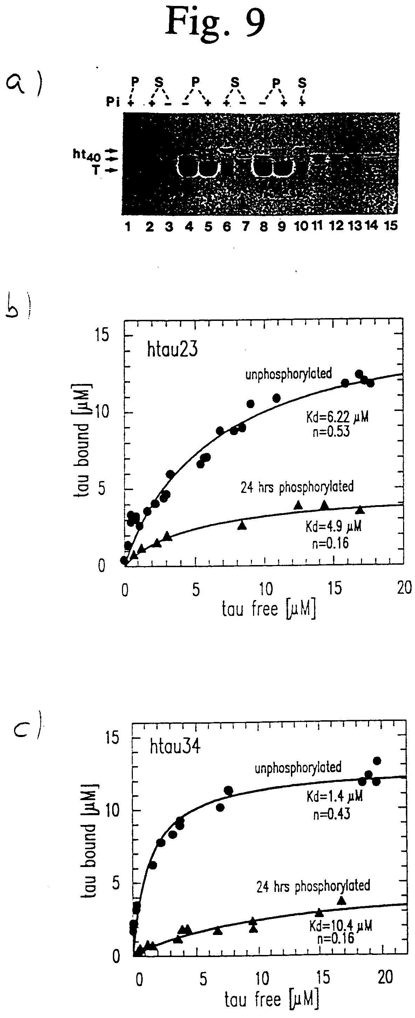

Fig. 9: Binding of tau isoforms to microtubules before and after phosphorylation.

(a) SDS gel of a binding experiment, illustrated for the case of the tau isoform htau40 (whose band is clearly separated from that of tubulin (T) so that both components can be shown simultaneously, without having to remove tubulin by a boiling step). The top line indicates pellets (P) or supernatants (S), with or without phosphorylation for 24 hours ( + or -$P_i$). Lanes 1-4, 20 $\mu$M tau protein (total concentration), phosphorylated (lanes 1, 2) or not (lanes 3, 4). The comparison of lanes 1 and 2 shows that most of the phosphorylated protein is free (S), while only a small fraction is bound to the microtubules (P). Lanes 3 and 4 show that in the unphosphorylated state about half of the protein is bound, the other half free (note also that the phosphorylated protein bands, lanes 1, 2, are higher in the gel than the unphosphorylated ones, lanes 3, 4, similar to Fig. 1). Lanes 5-8, similar experiment with 15 $\mu$M htau40. Lanes 9, 10 show the case of 10 $\mu$M phosphorylated protein. Lanes 11-15 are for density calibration with known amounts of htau40 (15, 10, 7.5, 5, and 2.5 $\mu$M, resp.).

(b) Binding curves of htau23 and (c) htau34 to microtubules before (circles) and after 24 hour phosphorylation (triangles); these curves were derived from SDS gels similar to that of Fig. 3a. Polymerized tubulin is 30 $\mu$M. Fitted dissociation constants $K_d$ and stoichiometries are as indicated. In each case the most dramatic effect is on the number of binding sites which decrease about three-fold upon phosphorylation, from around 0.5 (i.e. one tau for every two tubulin dimers) down to about 0.16 (one tau for six tubulin dimers). Note that the binding of unphosphorylated 4-repeat isoforms (such as htau34) is particulary tight ($K_d$ round 1-2 $\mu$M).

Fig. 10: Diagram of htau40, showing the location of the 7 ser-pro motifs phosphorylated by the kinase activity. The boxes labeled 1-4 are the internal repeats involved in microtubule binding; the second is absent in some isoforms (e.g. htau23). The two shaded boxes near the N-terminus are inserts absent in htau23 and htau24 so that these molecules have only 6 ser-pro motifs. The following radioactive tryptic peptides were found:

24- 49: KDQGGYTMHQDQEGDTDAGLKES$_P$PLQ

191-209: SGDRSGYSS$_P$PGS$_P$PGTPGSR

231-240: TPPKS$_P$PSSAK

396-406: SPVVSGDTS$_P$PR

386-406: TDHGAEIVYKS$_P$PVVSGDTS$_P$PR

407-428: HLSNVSSTGSIDMVDS$_P$PQLATL

260-266: IGS$_P$TENL

Fig. 11: Binding of htau34 to microtubules, before (circles) and after phosphorylation for 90 min (stage 1, triangles). The reduction in binding capacity is very similar to that after 24 hours phosphorylation (compare Fig. 9b).

Fig. 12: SDS-PAGE and immunoblots of tau protein from Alzheimer and normal human brain with antibodies SMI33, SMI31, and SMI34.

(a) Lane 1, SDS-PAGE of tau protein from a normal human control brain, showing 5-6 bands between $M_r$55 and 65 kD (somewhat lower than the PHF tau of lane 3). Lane 2, normal human tau after phosphorylation with kinase activity, resulting in an upward shift of all bands. Lanes 3, 4, immunoblot of PHF tau with antibody 5E2 which recognizes all tau isoforms independently of phosphorylation (Kosik et al., Neuron 1 (1988), 817-825). Lane 3, PHF tau as isolated from an Alzheimer brain; lane 4, after dephosphorylation with alkaline phosphatase. Note that the bands of the dephosphorylated protein are shifted down on the gel.

(b) Immunoblot of (a) with SMI33. The antibody recognizes normal human tau (lane 1), and PHF tau after dephosphorylation (lane 4).

(c) Immunoblot of (a) with SMI31. Note that the antibody recognizes normal human tau after phosphorylation, and PHF tau in its natural state of phosphorylation (lanes 2, 3).

(d) Immunoblot of (a) with SMI34. This antibody recognizes normal human tau only after phosphorylation (lane 2), and PHF tau (lane 3).

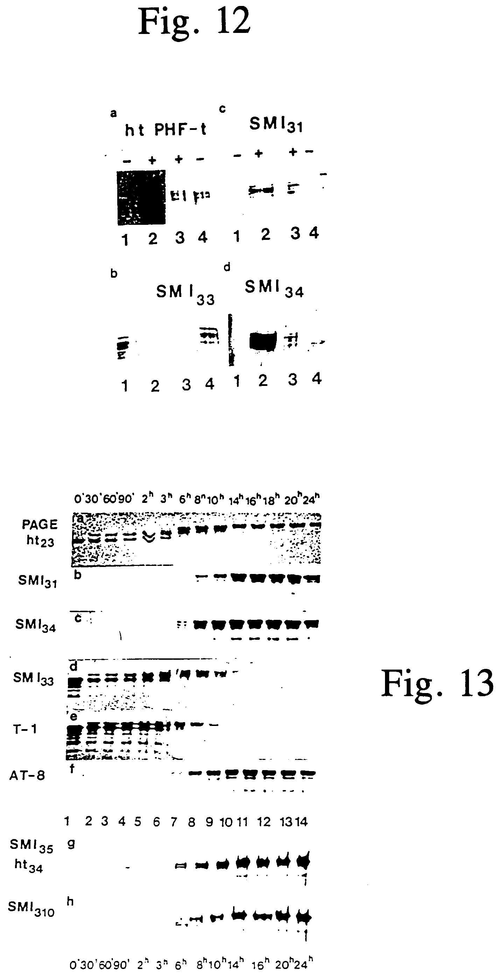

Fig. 13: Time course of phosphorylation of bacterially expressed human isoform htau23 (similar to previous figure) and immunoblots with antibodies SMI33, SMI31, SMI34, TAU1, and AT8.

(a) SDS-PAGE, phosphorylation times 0-24 hours, showing the successive $M_r$ shifts.

(b-f) Immunoblots with SMI31, SMI34, SMI33, TAU1, and AT8. Antibodies SMI33 and TAU1 recognize htau23 fully up to the end of stage 1 (2 hours), but the epitope becomes blocked during the second stage. Antibodies SMI31, SMI34, and AT8 are complementary in that they recognize the protein only in the second and third stage of phosphorylation.

(g-h) Immunoblot of htau34 with SMI33 and SMI310 which recognize the protein from the stage 2 phosphorylation onwards, similar to SMI31.

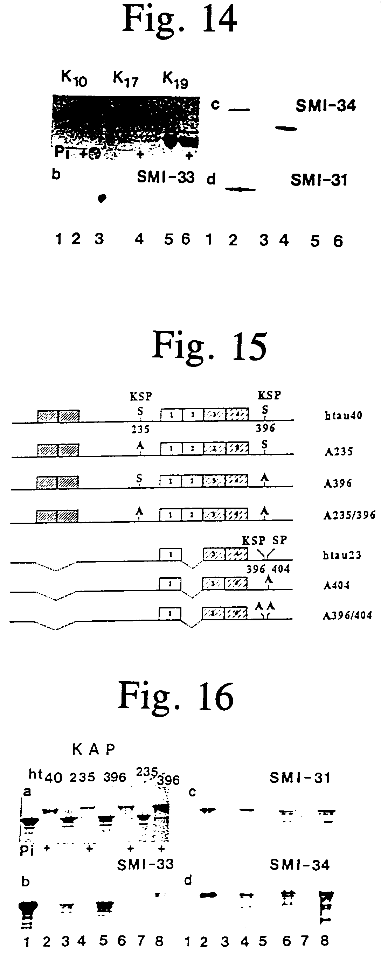

Fig. 14:    SDS-PAGE of tau and several constructs, and immunoblots with the antibodies SMI33, SMI31, and SMI34.

(a) SDS-PAGE. Lanes 1 and 2: Construct K10 before and after phosphorylation with the kinase for 24 hours. Lanes 3 and 4: Construct K17 before and after phosphorylation. Lanes 5 and 6: Construct K19 before and after phosphorylation. All constructs except K19 show a shift upon phosphorylation. With K10 one observes three shifted bands, with K17 there is only one shifted band.

(b) Immunoblot of (a) with SMI33: The antibody recognizes only K17 in the unphosphorylated form (lane 3), suggesting that the epitope lies before the repeats.

(c) Immunoblot of (a) with SMI34. The antibody recognizes K10 and K17 in the phosphorylated form (only top bands, lanes 2, 4). The antibody does not recognize K19 (the repeat region), but requires sequences on both the N-terminal and C-terminal side of the repeats. The epitope is therefore non-contiguous (conformation-dependent).

(d) Immunoblot of (a) with SMI31. The antibody recognizes only the top band of the phosphorylated K10 (lane 2), suggesting that the epitope lies behind the repeat region.

Fig. 15:    Diagram of point mutants of htau40 and htau23.

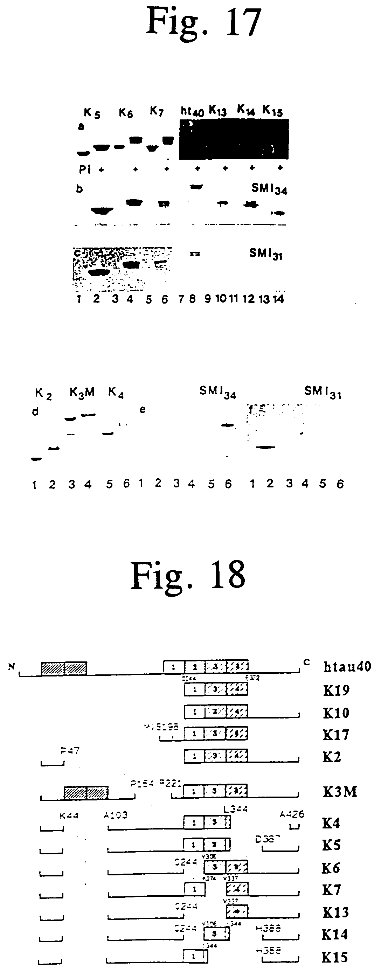

Fig. 16:    SDS gel of htau40 and the point mutants of Fig. 15, and immunoblots with antibodies SMI33, SMI31, and SMI34.

(a) Lanes 1-8, SDS gel of htau40 and its mutants KAP235, KAP396, and KAP235/396 in the unphosphorylated and phosphorylated form (+). In each case phosphorylation leads to an upward shift in the SDS gel.

(b) Blot of (a) with SMI33. The antibody response is strongly reduced when S235 is mutated, both in the dephosphorylated and phosphorylated state (lanes 3 + 4, 7 + 8). This indicates that the (dephosphorylated) first KSP motif is part of the epitope of SMI33. When S396 is mutated to A the behavior is similar to the parent molecule, i.e. strong antibody response in the dephosphorylated state, no reaction in the phosphorylated state, so that S396 does not contribute to the epitope of SMI33.

(c) Blot of (a) with SMI31. The antibody recognizes htau40 and all mutants in the phosphorylated form (lanes 2, 4, 6, 8). This shows that phosphorylation of the two KSP motifs is not the main determinant of the epitope.

(d) Blot of (a) with SMI34. The reaction is similar to SMI31 but more pronounced, again indicating that the two KSP motifs are not essential.

Fig. 17:    Deletion mutants of tau and their antibody response. (a) SDS gel of constructs containing only two repeats (K5-K7) or one repeat (K13-K15), before and after phosphorylation. (b) Immunoblot of (a) with SMI34. Note that the antibody recognizes all phosphorylated proteins (K7 only weakly). (c) Immunoblot of (a) with SMI31. Note that the antibody recognizes the phosphorylated two-repeat molecules (K5-K5), but not the one-repeat molecules (K13-K15). Lanes 7 and 8 show htau40 as a control. (d) SDS gel of constructs K2, K3M, and K4, before and after phosphorylation. (e) Blot of (d) with SMI34, recognizing only K4 phosphorylated. (f) Blot of (d) with SMI31, recognizing only K2 phosphorylated.

Fig. 18:    Diagram of htau40 and various mutants used in this study.

Example 1 Preparation of tau protein

Preparation of tau from normal brains: The procedures of tau preparation from human, bovine, or porcine brain, dephosphorylation, and rephosphorylation were essentially as described by Hagestedt et al., J. Cell. Biol. 109 (1989), 1643-1651.

Preparation of tau from Alzheimer brains: Human brain tissues from neuropathologically confirmed cases of Alzheimer's disease were obtained from various sources. The autopsies were performed between 1 and 25 hours post mortem. The brain tissue was kept frozen at -70°C. Tau from paired helical filaments (PHF) was prepared according to Greenberg & Davies, Proc. Natl. Acad. Sci. USA 87 (1990), 5827-5831.

Example 2 Characterization and partial purification of the tau phosphorylating activity (protein kinase) of porc brain extract

Porc brain extract supernatant was fractionated by ammonium sulphate precipitation. The main kinase activity precipitated at 40% saturation. This fraction was desalted by gel filtration, diluted fivefold and incubated in activation buffer (25 mM Tris, 2mM EGTA, 2mM DTT, 40mM p-nitrophenylphosphate, 10 $\mu$M okadaic acid, 2 mM MgATP, protease inhibitors) for 2 hours at 37°C. During this incubation a phosphorylation of a 44 kD protein at tyrosine residue(s) occurs as shown by Western blotting with anti-phosphotyrosine mAb. The 44 kD protein could be identified as MAP2 kinase by a second mAb.
The crude enzyme activity was further purified by ion exchange chromatography (Mono Q FPLC, Pharmacia). Fractions containing the activated MAP-Kinase, as shown by Western blotting, exerted the most prominent tau phosphorylating activity (Peak I). A second tau phosphorylating activity (Peak II) did not induce comparable SDS-gel shifts and Alzheimer-specific antibody reactivity in tau.

Example 3 Construction of plasmids carrying genes encoding recombinant tau polypeptides for the determination of Alzheimer tau protein specific epitopes

Cloning and expression of tau constructs: Plasmid preparations and cloning procedures were performed according to Sambrook et al. (Molecular Cloning Laboratory Handbook, 2nd edition, Cold Spring Harbor Laboratory, Cold Spring Harbor, 1989). Amplifications by the polymerase chain reaction (PCR, Saiki et al., Science 239 (1988), 487-491) were carried out using Tag polymerase as specified by the manufacturer (Perkin Elmer Cetus). The tau genes and their constructs were expressed in the expression vector pNG2, a derivative of pET-3b (Rosenberg et al., Gene 56 (1987), 125-135), modified by removal of PstI, HindIII, NheI and EcoRV restriction sites for convenient engineering of the tau gene. For the expression the BL21 (DE3) E. coli system (Studier et al., Meth. Enzym. 185 (1990), 60-89) was used. Most constructs were derived from the human isoform htau23 which contains 352 residues and three internal repeats in the C-terminal microtubule binding region (Goedert et al., proc. Natl., Acad. Sci. USA 85 (1988), 4051-4055). The numbering of residues used here refers to the sequence of htau40, the largest of the human isoforms (441 residues, Goedert et al., ibid.). For the isolation of the constructs use was made of the heat stability of the protein; they were separated by FPLC Mono S (Pharmacia) chromatography according to the procedure described by Hagestedt et al., J. Cell. Biol. 109 (1989), 1643-1651.
K10: This represents the carboxy part of the htau23 isoform consisting of 168 residues (Q244-L441 plus start methionine, but without the second repeat V275-S305). The K10 tau cassette was generated in the pNG2/htau23 vector by deletion of the NdeI-PstI fragment and replacing it with a chemically synthesized hexamer 5'TATGCA3'. After religation the NdeI endonuclease site was restored and PstI site was damaged.
K17: The K17 tau cassette (145 residues) is a shorter derivative of K16. It was made in two steps: First K16 was constructed using PCR to engineer the htau24 gene. The 5' "add on" of restriction sites on both ends of the amplified fragment was applied to facilitate the insertion of the PCR products into the cloning vector. The start primer (JB50) had the sequence GGCG ("G/C clamp"), the CATATG recognition site for the NdeI nuclease (containing the universal ATG start codon), followed by coding information for amino acids S198-T205. The stop primer (JB51) had a "G/C clamp" and the GGATCC recognition sequence for BamHI followed by a stop anticodon and anticoding sequence for the C terminal amino acids P364-E372. The K16 tau cassette consists of 176 residues, 175 from htau40 (S198-E372) plus a start methionine. This fragment represents part of the assembly domain consisting of 46 residues between S198 and the beginning of the first repeat following by the sequence of four repeats finished at E372. In the second step, a BstXI-BstXI fragment from the newly constructed tau K16 cassette was exchanged against the similar BstXI-BstXI fragment from the htau23 gene containing only three repeats and causing the generation of the tau cassette K17. Thus K17 represents the analogous part of the projection domain like K16 but missing the second tau repeat.
K3M (355 residues) is a chimera consisting of 145 residues from the amino terminus of bovine Tau4 (from the plasmid pETNde43-12, Himmler et al., Mol. Cell. Biol. 9 (1989), 1381-1388) and 190 residues from carboxy part of human htau23 (from the plasmid pUC18/htau23, Goedert et al., 1988 ibid.). It is a molecule with three repeats and two amino terminal inserts, consisting of 29 residues each. K3M was constructed by excision of XmaI-BclI fragment from pETNde42-12 and replacing it with analogous XmaI-BclI fragment originated from the htau23 gene. This manipulation removed 64 residues (XmaI-XmaI segment from bTau4) and replaced the 4 repeats carboxy terminus against three repeats carboxy terminus.

K19 represents the three repeats of htau23 and consists of 99 residues (Q244-E372, plus start methionine, without repeat 2). The K19 molecule was constructed from K17 by replacing the 144 nt long NdeI-PstI fragment with the synthetic hexamer 5'TATGCA3'. This modification retains the intact NdeI restriction site in the beginning of the molecule and removes the PstI site.

Construction of the D-mutant of htau23: In order to replace S199 and S202 by D in htau23, a double stranded DNA cassette encoding the amino acids G164-P219 was designed. This DNA segment was assembled from 8 oligonucleotides (30 to 60 nucleotides in length) and contained SfiI and XmaI sticky ends. The insertion of the assembled cassette into linearized pNG2/htau23 vector with removed native SfiI-XmaI fragment created the required gene.

Construction of htau23/A404: htau23/A404 is a mutated htau23 molecule where Ser404 was replaced by the Ala in order to remove this phosphorylation site. For convenient manipulation of the htau23 gene, an artificial NcoI restriciton site in the position 1161 (htau40 numbering) was introduced. This mutation was done using PCR-SOE (splicing by overlap extension, Higuchi et al., Nucl. Acids. Res. 16, (1988), 7351-7367). The new NcoI does not influence the amino acid sequence of tau protein. For the introduction of the Ala residue in the position 404 a synthetic DNA cassette was used, representing the 120 bp DNA fragment between NcoI and NheI restriction sites and encoding the amino acids His388-Thr427. This DNA segment was assembled from 4 oligonucleotides (54 to 66 nucleotides in length) and contained NcoI and NheI sticky ends. The insertion of the assembled cassette into the linearized pNG2/htau23/NcoI vector with removed native NcoI-NheI fragment created the htau23/A404 gene. The mutation of Ser396 to Ala was created in similar way like that in the position 404.

K2 (204 residues) is a chimera consisting of 36 residues from the amino terminus of bovine Tau4 and 168 residues from the carboxy part of htau23; it contains three repeats. K4-K7 are deletion mutants of htau23 containing only two repeats: K4 has repeats No. 1 and 3 (270 residues, D345-A426 excised); K5 has repeats No. 1 and 3 (310 residues, D345-T386 excised); K6 has repeats No. 3 and 4 (322 residues, T245-K274 excised); K7 has repeats No. 1 and 4 (321 residues, V306-Q336 excised); note that repeat No. 2 is always absent in htau23. K13-K15 are deletion mutants of htau23 containing only one repeat: K13 has repeat No. 4 (291 residues, T245-Q336 excised); K14 has repeat No. 3(279 residues, T245-S305 and D345-D387 excised); K15 has repeat No. 1 (278 residues, D345-D387 excised).

Example 4 Determination of Alzheimer specific epitope in the tau protein

A panel of antibodies against PHFs from Alzheimer brain was closely examined for their reactivity and one (AT8) was found that was specific for PHF tau. Fig. 1 shows the reactivity of the antibody AT8 against different tau species. In the case of tau from Alzheimer paired helical filaments (PHF) the antibody recognizes all isoforms (Fig. 1b, lane 1). When the mixture of tau isoforms from normal bovine or human brain was tested (known to be in a mixed state of phosphorylation, Fig. 1a, lanes 2-5), reactivity with the AT8 antibody (Fig. 1b) was detected. The same is true for the six individual human isoforms expressed in E. coli (unphosphorylated, Fig. 1a and 1b, lanes 6-11). It is concluded that AT8 is indeed specific for Alzheimer tau; in particular, it reacts with a phosphorylated epitope that occurs only in PHFs, but not in normal tau. Moreover, there is a correlation between the AT8 reactivity, phosphorylation, and electrophoretic mobility; it appears as if there was an Alzheimer-like phosphorylation that caused an upward shift in the SDS gel.

In order to identify the kinase(s) that were responsible for this behavior, and the corresponding phosphorylation sites, a kinase activity from porcine brain extract was prepared as described in Example 2. The six human isoforms expressed in E. coli were phosphorylated according to standard procedures with this activity in the presence of okadaic acid, a phosphatase inhibitor. Fig. 2a shows that each isoform changes considerably its electrophoretic mobility in the gel (upward shift) and shows a strong immunoreactivity with the AT8 antibody (Fig. 2b). These results show that the phosphorylation of tau by this kinase activity is analogous to that of the Alzheimer state. Moreover, since all isoforms are affected in a similar way the phosphorylation site(s) must be in a region common to all of them.

The strategy to identify said common region was to use first the engineered mutants prepared as described in Example 3 in order to narrow down the site, and then to determine it by direct sequencing. Fig. 3 describes some of the mutants used, K19, K10, K17, and K3M (see also Example 3). Except for K19, all of these mutants are phosphorylated by the kinase activity and show an upward M shift in the SDS gel (Fig. 4a). K19 is a construct that comprises just three repeats of 31 or 32 residues. It does not become phosphorylated by the kinase activity and therefore does not show an $M_r$ shift in the SDS gel (Fig. 4c).

This means that the phosphorylation site(s) are outside the region of the repeats. Phosphorylation can take place on either side of the repeats and induces an upward shift in the gel; the shift is larger for

11

phosphorylation after the repeats. The antibody AT8 recognizes none of the unphosphorylated forms (as expected); after phosphorylation it reacts only with the construct K17 (Fig. 4b, lane 6), not with K10 or K3M (Fig. 4b, lanes 4 and 8). In other words, K17 retains the epitope, while K10 and K3M have lost it. By reference to Fig. 3 it is concluded that the epitope is not in the region of the pseudo-repeats nor in C-terminal tail where we found a CaM kinase site previously (since K10 and K19 are non-reactive), but rather it has to be between S198 and T220 (Fig. 3, peptide P), i.e. in the region following the major chymotryptic cleavage site (behind Y197) in the "assembly" domain of tau.

Next a total tryptic digest of radioactively labeled htau34, an isoform with 4 internal repeats (Goedert et al., 1989, ibid.) was carried out. The peptides were isolated by HPLC and sequenced. One of them was in the area of interest, S195-R209 (Fig. 5). This peptide contained two phosphates at S199 and S202. Both are followed by a proline, suggesting that the enzyme active in the extract was a proline-directed kinase.

These results suggested that the phosphorylation sensitive AT8 epitope might be in the vicinity of residue 200. This was tested by engineering a mutant of htau22 (3 repeats, no N-terminal insert) where S199 and S202 were both changed to D. This choice was made in order to rule out the phosphorylation of these residues by a kinase, but also to mimick in part the "phosphorylated" state in terms of negative charges. On SDS gels this mutant showed a small upward shift to higher M (Fig. 6, lane 4). The immunoblots show that only the parent protein htau23 reacted with the antibody after phosphorylation (Fig. 6, lane 6), but not the unphosphorylated htau23 (as expected) nor the mutant, whether phosphorylated or not (lanes 7, 8).

It is concluded that the epitope of AT8 is in the region S199-S202 and depends on the phosphorylation of these two serines. They can be phosphorylated by a proline-directed kinase present in brain extract which turns the protein into an Alzheimer-like state. The region is perfectly conserved in all tau variants known so far and explains why all of them respond to phosphorylation and to the antibody in the same way.

Example 5 Characterization of the protein kinase activity

Phosphorylation of tau proteins was carried out in the following way: Tau protein (0.5 mg/ml) was incubated for various times (up to 24 hours) at $36°C$ with the brain extract in 40 mM HEPES containing 2 mM $MgCl_2$, 1 mM DTT, 5 mM EGTA, 1.5 mM PMSF, 2 mM ATP, 20 $\mu$g/ml protease inhibitor mix (pepstatin, leupeptin, alpha-macroglobulin, aprotinin), with or without 1 mM okadaic acid. After that 500 mM DTT were added, the solution was boiled for 10 min and centrifuged for 15 min at 15000 g at $4°C$. The supernatant was dialyzed against reassembly buffer (RB, 100 mM Na-PIPES pH 6.9, 1 mM EGTA, 1 mM GTP, 1 mM $MgSO_4$, 1 mM DTT) and used for binding studies.

Radioactive labeling was done with gamma-[$^{32}$P]ATP (NEN Du Pont) at 10 mCi/ml, 3000 Ci/mmol, diluted to 15-30 Ci/mol ATP for autoradiography on SDS gels. The phosphate incorporated into the protein was quantified as follows: 1 $\mu$g of phosphorylated protein was applied to SDS gels, the bands were cut out and counted in the scintillation counter in Cerenkov mode. The counter was calibrated with known samples of $^{32}$P (detection efficiency about 50% in Cerenkov mode). The corrected counts were translated into moles of $P_i$ per mole of tau on the basis of the known specific activity of radioactive ATP used during phosphorylation.

A remarkable feature found for this kinase is that it shifts the $M_r$ of all tau isoforms in three distinct stages (see Fig. 7a and 8a for the case of htau23). During the first two hours of phosphorylation the protein is converted from a $M_{ro}$ = 48 kD protein to a slower species, with an $M_{r1}$ of about 52 kD. Upon completion of this first stage, a second one sets in which is finished aroumd 6.10 hours ($M_{r2}$ = 54 kD), The third stage takes about 24 hours ($M_{r3}$ = 56 kD), after that no more shift is observed.

During the initial stage each band of the tau doublet incorporates phosphate (e.g. at a level of about 0.5 $P_i$ per molecule in the presence of OA at 30 min, see Fig. 7b, lane 4). This means that there must be at least two distinct phosphorylation sites, one that is responsible for the shift (the "shift site", upper band), and one that has no effect on the $M_r$ (lower band). The lower band gradually disappears, and at two hours each tau molecule contains about 2 $P_i$. In other words, the upper band contains tau molecules in which the "shift site" is phosphorylated, irrespective of the other site(s); whereas the lower band contains only molecules where the shift site is not phosphorylated. The effect of OA is seen mainly in the lower band, indicating that the phosphatase operates mainly on the non-shift site(s). These considerations apply to the first stage of phosphorylation; during the second and third stages there are further shifts, but a detailed analysis of shift sites and non-shift sites is not possible because of the overlap of bands. Overall about two additional phosphates can be incorporated in every stage, giving a maximum of 6 for htau23 and 7 for htau34. These values refers to the presence of OA; without it we usually find ~1-2 $P_i$ less. When the purified kinase is used, one finds 12-14 $P_i$.

Since the major shift occurs during the first stage, and since a large shift is considered a hallmark of Alzheimer tau, it was suspected that the first stage phosphorylation might induce an Alzheimer-like state. This was checked by immunoblotting according to standard procedures with Alzheimer-specific antibodies. Fig. 8a shows a similar phosphorylation experiment as above (with 10$\mu$M OA throughout), Fig 8b is the immunoblot with the monoclonal antibody SMI34 which reacts with a phosphorylated epitope in Alzheimer tangles (Sternberger et al., ibid.) The antibody recognizes the bacterially expressed tau phosphorylated by the kinase, but only from stage 2 onwards. A similar behavior is found with other Alzheimer-specific antibodies tested. The result from these studies is that the major phosphorylation-dependent $M_r$ shift (stage 1) is distinct from the ones that generate the Alzheimer-like antibody response (stages 2, 3).

Example 6 Tau protein in microtubule binding studies.

Another point of interest with respect to the correlation between abnormal phosphorylation of tau proteins and Alzheimer disease was whether the phosphorylation had an influence on tau's affinity for microtubules. This was tested using a microtubule binding assay. Accordingly, PC tubulin was incubated at 37°C in the presence of 1 mM GTP and 20 $\mu$M taxol. After 10 min tau protein was added in different concentrations and incubated for another 10 min. The suspensions were centrifuged for 35 min at 43000 g at 37°C. The resulting pellets were resuspended in CB buffer (50 mM PIPES PH 6.9, 1 mM EGTA, 0.2 mM $MgCl_2$, 5 mH DTT, 500 mH NaCl). In the case of htau 23 and htau 34 the pellets and supernatants were boiled for 10 min and recentrifuged for 10 min at 43000 g at 4°C (this step served to remove the tubulin component which otherwise would overlap with these tau isoforms on SDS gels). Pellets and supernatants (containing the bound and the free tau, respectively) were subjected to SDS PAGE (gradient 7-15 % acrylamide) and stained with Coomassie brilliant blue R250. The gels were scanned at 400 dpi on an Epson GT 6000 scanner and evaluated on a PC 368AT using the program GelScan (G. Spieker, Aachen). The protein concentration on the gel was always within the linear range (up to 1.5 optical density). The intensities were transformed to concentrations using calibration curves and used in the binding equation.

$Tau_{bound} = n[Mt][Tau_{free}]/\{Kd + [Tau_{free}]\}$, from which the dissociation constand $K_d$ and the number n of binding sites per dimer were obtained by fitting. [Mt] is the concentration of tubulin dimers polymerized in microtubules (usually 30 $\mu$M).

With fully phosphorylated protein (stage 3, 24 hours) a dramatic decrease in binding capacity of htau23 was observed (Fig 9b), from about one tau per two tubulin dimers to one tau per six tubulin dimers. In other words, it appears that unphosphorylated tau packs tightly onto a microtubule surface, whereas fully phosphorylated tau covers the microtubule surface less densely, as if it occupied more space. Fig. 9c shows the same experiment with htau34. The results are similar, i.e. there is a threefold reduction in binding capacity. Tau isoforms with four repeats, such as htau34, bind to microtubules particularly tightly in the unphosphorylated state ($K_d$~1-2 $\mu$M).

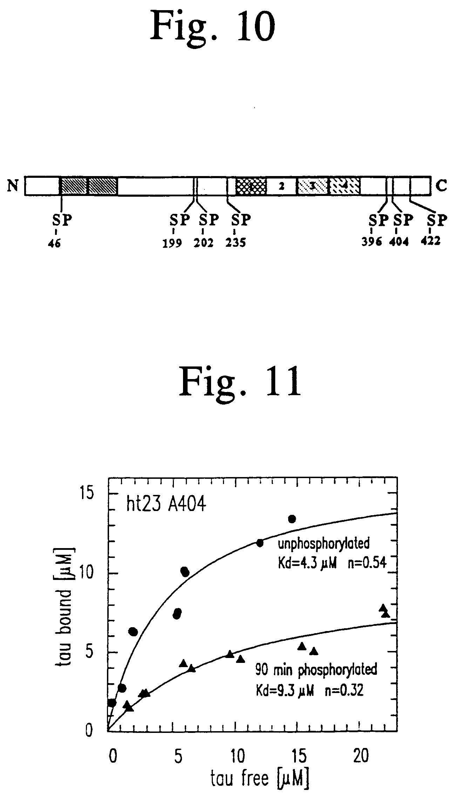

Since the major $M_r$ shift (see Example 5) occurs during the initial two hours it was of interest to find out which residues become phosphorylated during the first stage, and how they affected microtubule binding. As mentioned above, there are about two phospates incorporated during this period, one of which causes the shift from $M_{ro}$ to $M_{r1}$. Fig. 10 illustrates the binding of htau34 to microtubules after 90 min of phosphorylation. The striking result is that the limited phosphorylation decreases the affinity as efficiently as the full phosphorylation. This means that the reduction in microtubule affinity precedes the Alzheimer-like immunoreactivity (Fig. 8).

The analysis of tryptic peptides after 90 min showed four major peaks of radioactivity, with phosphates on serines 202, 235, 404, and 262. Three of these are SP sites that are not in the repeat region, but rather flank that region in nearly symmetric positions (Fig. 11); the fourth (S262) is a non-SP site in the first repeat. It is in particular note-worthy that S396 was not among the phosphorylated residues. This was unexpected since Lee et al. (1991, ibid.) had shown that S396 (the center of a KSP motif) was phosphorylated in tau from paired helical filaments. Thus S396 must become phosphorylated during the second or third stages of phosphorylation, concomitant with the immunoreactivity (Fig. 8b).

Several point mutants were generated according to standard procedures to find out which site(s) were responsible for the initial $M_r$ shift. When ser404 was turned into ala the $M_r$ shift during the first stage disappeared, whereas it remained visible when ser199, 202, 235, or 396 were mutated. This means that the phosphorylation of ser404 accounts for the one $P_i$ present in the upper band of Fig. 7a or 8a. The additional ~1$P_i$ present after 2 hours is distributed among serines 202, 235, and 404.

Whereas the results on the "shift site" S404 of tau are clear cut, the factors responsible for the reduction of microtubule binding are more complex. The S404-A mutant binds to microtubules similarly as the parent htau34; after 90 min of phosphorylation the stoichiometry decreases about 2-fold, i.e. less than the factor of 3 observed with the parent molecule. If S404 were the only residue whose phosphorylation was responsible for the loss of microtubule binding we would not expect any decrease in the mutant. The fact that a decrease is observed means that other factors play a role as well; these factors are presumably related to the incorporation of more than one $P_i$ at one or more of the other sites before or at the beginning of the repeat region (e.g. 202, 235, 262). However, these residues cannot by themselves be responsible for the full decrease of affinity either. In fact, point mutations at positions 202 or 235 show a similar effect as that of 404, i.e. only a partial reduction of binding. One possible explanation is that different phosphorylation sites interact in a cooperative manner and generate a new confirmation.

Example 7 Time course of phosphorylation as determined by stage specific antibodies

Neurofilament specific antibodies SMI31, SMI34, SMI35 and SMI310 against a phosphorylated epitope and SMI33 against a non-phosphorylated epitope [(Sternberger et al., Proc. Natl. Acad. Sci. USA 82 (1985), 4274-4276)] were used to detect stage specific phosphorylation of tau protein. SMI33 recognizes normal human brain tau (Fig. 12, lane 1) but does not recognize PHF tau, except when it is dephosphorylated (lane 4). This suggests that the epitope of SMI33 is specifically blocked by some phosphorylation in the Alzheimer state which does not occur in normal brain tau. SMI31 and SMI34 both react in a complementary fashion to SMI33: Only PHF tau is recognized (Fig. 12c and 12d, lane 3), but not when it is dephosphorylated (lane 4), nor the normal tau control (lane 1).

The testing of the various antibodies during the time course of phosphorylation shows that SMI33 loses reactivity during the second stage of phosphorylation (see Fig. 7).

For antibody SMI31 no reactivity is observed with the unphosphorylated protein (time 0) or during the first stage, but the reactivity appears gradually during the second stage and remains throughout the third. A similar time course is found with antibody SMI34 (Fig. 13c and compare Fig. 12d, lane 3), SMI35, and SMI310 (Fig. 13g,h). For comparison the blots with AT8 (Fig. 13f), a phosphorylation sensitive Alzheimer tangle antibody (Binder et al., J. Cell. Biol. 101 (1985), 1371-1379 are included) and TAU1, an antibody against dephosphorylated tau. AT8 reacts similarly to SMI31, SMI34, SMI35, and SMI310, while TAU1 is similar to SMI33. The striking feature of the blots is that in each case it is the stage 2 phosphorylation that determines the antibody response.

These experiments could be interpreted by assuming that the antibodies react with the same region of tau in a dephosphorylated or phosphorylated form; but this assumption is too simple, as shown later. Two other features should be pointed out, however: One is that the largest gel shift (stage 1) is not the one that causes the Alzheimer-like immunoreactivity (appearing in stage 2). Thus not every gel shift of tau is diagnostic of the Alzheimer state, although conversely the Alzheimer state always shows a gel shift. Secondly, there is a surprisingly precise relationship between gel shift, phosphorylation, and immunoreactivity with several different antibodies.

The major phosphorylated motifs of neurofilaments are repeated sequences of the type KSPV where S is the phosphate acceptor; see e.g. Geisler et al., FEBS Lett. 221 (1987), 403-407. Tau has one such motif, centered at S396, and another KSP motif is centered at S235. The two KSP sites lie on either side of the repeat region and are conserved in all tau isoforms. By analogy one may suspect that these sites are involved in the reaction with the SMI antibodies that were raised against neurofilaments. We tested this in three ways, by mutating one or two of the serines, by making smaller tau constructs, and by direct sequencing of tryptic peptides.

Constructs K10, K17, and K19 were examined before or after phosphorylation with the kinase (Fig. 14a). K10 and K17 show an $M_r$ shift, but not K19. Note also that K10 and K17 are only partly converted to the higher $M_r$ form in this experiment, indicating that their phosphorylation is less efficient. K10 shows three shifted bands, indicating that there are three phosphorylation sites in the C-terminal region. K17 shows only one shifted band so that there is only one shift-inducing site in the region before the repeats. Fig. 14b-d show the immunoblots with SMI33, SMI31, and SMI34; the data on SMI35 and SMI310 are similar to SMI31 (not shown). Antibody SMI33 reacts only with K17 in the dephosphorylated state, but not with K10 and K19 (Fig. 14, lane 3). This suggests that the epitope is in a region before the repeats, between S198 and Q244, outside the sequences covered by the other constructs. This would be consistent with an epitope at the first KSP site. Antibody SMI31 reacts with K10 in its phosphorylated form, but not K17 or K19 (Fig. 14). Using similar arguments as before, the epitope is in the region T373-L441, consistent with the second KSP site. Finally, antibody SMI34 labels htau23, K10 and K17, but not K19 (Fig. 14c). The latter property would argue against the repeat region as an epitope, but the remaining reaction with K10 and K17 would seem mutually exclusive. Our interpretations is that SMI34 has a conformational epitope that depends on tails on either side of the repeats and becomes fully stabilized only when at least one tail is present. However, the phosphorylation dependence is in each case the same as that of the intact molecule.

Since it was suspected that the two KSP motifs were phosphorylated by the kinase, it was tried to prove this directly. Radioactively labeled tryptic peptides of htau34 were identified by HPLC and protein sequencing, and phosphorylated residues were determined. There are two major phosphorylated tryptic peptides in these regions; peptide 1 (T231-K240, TPPKS$_p$PSSAK) contains the first KSP motif, phosphorylated at S235, peptide 2 (T386-R406, TDHGAEIVYKS$_p$PVVSGDTS$_p$PR) contains the second KSP site, phosphorylated at S396 and S404. S416, the single phosphorylation site of CaM kinase described earlier (Steiner et al. EMBO J. 9 (1990), 3539-3544, S405 in the numbering of htau 23 used earlier) is not phosphorylated by the kinase used here.

Next point mutants of the phosphorylated residues 235 and 396 (Fig. 15) were made and analysed in terms of gel shift and antibody reactivity (Fig. 16). The parent protein htau40 and its KAP mutants have nearly identical $M_r$ values, and they all shift by the same amount after phosphorylation (Fig. 16, lanes 1-8). The reactivity of SMI33 is strongly reduced when S235 is mutated to A (Fig. 16, lanes 3, 7) and obliterated after phosphorylation (Fig. 16, lanes 2, 4, 6, 8). This means that the epitope of SMI33 is around the first KSP site, but phosphorylation at other sites have an influence as well (perhaps via a conformation). The mutation at S396 (second KSP site) has no noticeable influence on the SMI33 staining (Fig. 16b, lanes 5, 6).

As mentioned above, the epitope of SMI31 depends on the phosphorylation of sites behind the repeat region. When S396 is mutated to Ala the antibody still reacts in phosphorylation dependent manner so that this serine is not responsible for the epitope by itself (Fig. 16c, lane 6). Mutation S404 to Ala yields the same result. However, if both serines are mutated, the antibody no longer reacts upon phosphorylation (not shown). This means that the epitope includes the two phosphorylated serines. The binding of this antibody also has a conformational component: constructs with only one repeat (K13-K15) are not recognized (Fig. 17, lanes 10, 12, 14).

SMI34 shows the most complex behavior because its reactivity depends on phosphorylation sites before and after the repeat region. This antibody recognizes all KAP mutats, so that S235 and S396 cannot play a major role. However, the fact that SMI34 recognizes phosphorylated K17, K10, but not K19 (Fig. 17) suggests that the regions before and/or behind the repeats must cooperate with the repeats to generate the epitope. One possibility would be that the epitope is non-contiguous, another one is that it may depend on the number and conformation of the repeats. In order to check these possibilities constructs with different combinations of two repeats (K5, K6, K7, Fig. 18), and constructs with one repeat only (K13, K14, K15) were done. All of these showed a shift upon phosphorylation, and all of them were recognized by SMI34 (the reaction is less pronounced when the third repeat is absent, indicating that this repeat is particularly important for the conformation, Fig. 17, lane 6). This means that the epitope of SMI34 does not depend on the number of repeats. However, the nature of the region just before the repeats seems to be important and in particular sensitive to charges. This can be deduced from constructs such as K2 or K3M where charged sequences have been brought close to the repeat region, resulting in a loss of SMI34 reactivity. In other words, it seems as if the charged sequences are capable of masking the epitope, independently of the phosphorylation itself (Fig. 17, lanes 2, 4). The interactions between the constructs and the antibodies are summarized in Table 1.

SEQUENCE LISTING

| SEQ. ID No. | Residue Nos. | Sequence (single letter code) | Sequence type | Sequence length | Strandedness |
|---|---|---|---|---|---|
| 1 1 | 44- 49 | K E S P L Q<br>LysAspSerProLeuGln | amino acid | 6 | single |
| 2 2 | 197-203 | Y S S P G S P<br>TyrSerSerProGlySerPro | " " | 7 | " |
| 3 3 | 200-205 | P G S P G T<br>ProGlySerProGlyThr | " " | 6 | " |
| 4 4 | 197-208 | Y S S P G S P G T P G S<br>TyrSerSerProGlySerProGlyThrProGlySer | " " | 12 | " |
| 5 5 | 233-238 | P K S P S S<br>ProLysSerProSerSer | " " | 6 | " |
| 6 6 | 394-400 | Y K S P V V S<br>TyrLysSerProValValSer | " " | 7 | " |
| 7 7 | 401-407 | G D T S P R H<br>GlyAspThrSerProArgHis | " " | 7 | " |
| 8 8 | 419-425 | M V D S P Q L<br>MetValAspSerProGlnLeu | " " | 7 | " |
| 9 9 | 47- 53 | P L Q T P T E<br>ProLeuGlnThrProThrGlu | " " | 7 | " |
| 10 10 | 43- 54 | L K E S P L Q T P T E D<br>LeuLysAspSerProLeuGlnThrProThrAspAsp | " " | 12 | " |
| 11 11 | 66- 72 | A K S T P T A<br>AlaLysSerThrProThrAla | " " | 7 | " |
| 12 12 | 108-114 | I G D T P S L<br>IleGlyAspThrProSerLeu | " " | 7 | u |
| 13 13 | 172-178 | P A K T P P A<br>ProAlaLysThrProProAla | " " | 7 | " |
| 14 14 | 178-184 | A P K T P P S<br>AlaProLysThrProProSer | " " | 7 | " |
| 15 15 | 172-184 | P A K T P P A P K T P P S<br>ProAlaLysThrProProAlaProLysThrProProSer | " " | 13 | " |
| 16 16 | 202-208 | S P G T P G S<br>SerProGlyThrProGlySer | " " | 7 | " |
| 17 17 | 209-215 | R S R T P S L<br>ArgSerArgThrProSerLeu | " " | 7 | " |
| 18 18 | 214-220 | S L P T P P T<br>SerLeuProThrProProThr | " " | 7 | " |
| 19 19 | 209-220 | R S R T P S L P T P P T<br>ArgSerArgThrProSerLeuProThrProProThr | " " | 12 | " |
| 20 20 | 228-234 | V V R T P P K<br>ValValArgThrProProLys | " " | 7 | " |
| 21 21 | 228-239 | V V R T P P K S P S S A<br>ValValArgThrProProLysSerProSerSerAla | " " | 12 | " |

SOURCE: Peptides of tau protein determined by sequencing (see text).

FEATURES: All Ser-Pro or Thr-Pro motifs are of potential diagnostic value for Alzeimer's disease, with Ser, Thr either in the phosphorylated state or in the dephosphorylated state.

## Claims

1. An epitope of the tau protein which is specifically occurring in a phosphorylated state in tau protein from Alzheimer paired helical filaments.

2. The epitope according to claim 1 which is specifically phosphorylated by a protein kinase from mammalian brain having the following biochemical properties:

(a) It phosporylates ser-pro and thr-pro motifs in tau protein;

(b) it has an $M_r$ of 42 kD;

(c) it is activated by ATP and has a $K_m$ of 1.5 mM;

(d) it is activated by tyrosine phosphorylation;

(e) it is recognized by an anti-MAP kinase antibody.

3. The epitope according to claim 1 or 2 which includes the phosporylatable serine residues 46, 199, 202, 235, 296, 404 and/or 422 and/or the phosphorylatable threonine residues 50, 69, 111, 175, 181, 205, 212, 217 and/or 231.

4. The epitope according to any one of claims 1 to 3 which is an epitope comprising the amino acid sequences

KESPLQ, YSSPGSP, PGSPGT, YSSPGSPGTPGS, PKSPSS, YKSPVVS, GDTSPRH, MVDSPQL; PLQTPTE, LKESPLQTPTED, AKSTPTA, IGDTPSL, PAKTPPA, APKTPPS, PAKTPPAPKTPPS, SPGTPGS, RSRTPSL, SLPTPPT, RSRTPSLPTPPT, VVRTPPK, VVRTPPKSPSSA.

5. A protein kinase which is capable of specifically converting tau protein to Alzheimer tau protein by phosphorylation of the amino acid motif ser-pro or thr-pro.

6. The protein kinase according to claim 5 which has the following biochemical properties

(a) It phosporylates ser-pro and thr-pro motifs in tau protein;

(b) it has an $M_r$ of 42 kD;

(c) it is activated by ATP and has a $K_m$ of 1.5 mM;

(d) it is activated by tyrosine phosphorylation;

(e) it is recognized by an anti-MAP kinase antibody.

7. The protein kinase according to claim 5 or 6 which is obtainable by

(a) homogenizing porcine brain in 10 mM Tris-HCl, pH 7,2, 5 mM EGTA, 2 mM DTT and a cocktail of protease inhibitors (leupeptin, aprotinin, pepstatin A, α2-macroglobulin, PMSF);

(b) centrifugating the homogenate at 100,000 x g for 30 minutes at 4°C;

(c) removing the supernatant after centrifugation;

(d) precipitating the crude protein by ammonium sulfate precipitation;

(e) desalting the crude preparation by gel filtration;

(f) activating the crude enzyme by incubation in activation buffer;

(g) further purifying the crude preparation by ion exchange chromatography; and

(h) identifying the enzyme by Western blotting.

8. The protein kinase according to any one of claims 5 to 7 which specifically phosphorylates serines 46, 199, 202, 235, 396, 404, 422 and threonines 50, 69, 111, 175, 181, 205, 212, 217, 231 of the tau protein.

9. The protein kinase according to any one of claims 5 to 8 which is a protein kinase from human brain, porcine brain, or another source.

10. A pharmaceutical composition containing a specific inhibitor for the protein kinase of any one of claims 5 to 9, optionally in combination with a pharmaceutically acceptable carrier and/or diluent.

11. The pharmaceutical composition according to claim 10 for use in the treatment of Alzheimer disease.

12. The pharmaceutical composition according to claim 10 or 11 which contains as the specific inhibitor at least one oligo- or polypeptide comprising an epitope according to any one of claims 1 to 4.

13. An antibody which specifically recognizes an epitope according to any one of claims 1 to 4.

**14.** An antibody which specifically recognizes the protein kinase according to any one of claims 5 to 9.

**15.** The antibody according to claim 13 or 14 which is a monoclonal antibody.

**16.** A diagnostic composition for the detection and/or monitoring of Alzheimer disease comprising:
- an epitope according to any one of claims 1 to 4;
- a kinase according to any one of claims 5 to 9;
- an antibody according to claim 13 or 14; and/or
- an antibody according to claim 15 or 16.

**17.** A method for the in vitro diagnosis and/or monitoring of Alzheimer disease comprising assaying a cerebrospinal fluid isolate of a patient or carrying out a biopsy of nerve tissue
- for the presence of a phosphorylated Alzheimer tau protein containing an epitope according to any one of claims 1 to 4 or
- for the presence of a protein kinase according to any one of claims 5 to 9.

**18.** The method according to claim 17, wherein the Alzheimer tau protein is detected by using an antibody according to claim 13.

**19.** The method according to claim 17, wherein the protein kinase is detected by using an oligo or polypeptide comprising an epitope according to any one of claims 1 to 4 and by using an antibody according to claim 13 or 15.

**20.** A method for the in vitro conversion of the tau protein into Alzheimer tau protein wherein normal tau protein is treated with a protein kinase of any one of claims 5 to 9 under conditions which allow the phosphorylation of said normal tau protein.

**21.** Use of an epitope according to any one of claims 1 to 4 for the generation of Alzheimer tau protein specific antibodies.

TABLE 1

| Interactions of tau constructs with antibodies in the phosphorylated or unphosphorylated state ( + or -). The staining on immunoblots ranges from very weak, (x), to very strong, xxx. | | | | |
|---|---|---|---|---|
| construct | phosph. +/- | SM133 | SM131 | SM134 |
| htau40 | - | xxx | | |
| | + | | xxx | xxx |
| htau23 | - | xxx | | |
| | + | | xxx | xxx |
| K3M | - | | | |
| | + | | (x) | |
| K2 | - | | | |
| | + | | xxx | |
| K17 | - | xxx | | |
| | + | | | xx |
| K10 | - | | | |
| | + | | xxx | xxx |
| K19 | - | | | |
| | + | | | |
| htau40/A235 | - | (x) | | |
| | + | | xxx | xxx |
| htau40/A396 | - | xxx | | |
| | + | | xx | xxx |
| htau40/A235/A396 | - | (x) | | |
| | + | | xx | xxx |
| htau23/A404 | - | xxx | | |
| | + | | xxx | xxx |
| htau23/A396/A404 | - | xxx | | |
| | + | | | xxx |
| K4 | - | xxx | | |
| | + | | | xx |
| K5 | - | xxx | | |
| | + | | xx | xxx |
| K5 | - | xxx | | |
| | + | | xx | xxx |
| K7 | - | xxx | | |
| | + | | x | xx |
| K13 | - | xxx | | |
| | + | | | xx |
| K14 | - | xxx | | |
| | + | | | xx |
| K15 | - | xxx | | |
| | + | | | xx |

Fig. 1

# Fig. 2

Fig. 3

Fig. 4

htau 23

Fig. 5

Fig. 6

# Fig. 7

# Fig. 8

# Fig. 9

a)

b)

c)

# Fig. 10

# Fig. 11

# Fig. 12

# Fig. 13

# Fig. 14

# Fig. 15

# Fig. 16

# Fig. 17

# Fig. 18

European Patent
Office

**EUROPEAN SEARCH REPORT**

Application Number

EP    91 12 0974

Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X,D | SCIENCE. vol. 896, no. 4994, 8 February 1991, LANCASTER, PA US pages 675 - 678; LEE, V.M.Y. ET AL.: 'A68 : A major subunit of paired helical filaments and derivatized forms of normal tau' * abstract * * page 677, column 2 - page 678, column 1 * --- | 1,3,4, 13, 16-18,21 | C12N15/12 C12N9/12 A61K37/02 C12P21/08 G01N33/68 C07K7/00 A61K37/64 |
| X | PROCEEDINGS OF THE NATIONAL ACADEMY OF SCIENCES OF USA. vol. 85, June 1988, WASHINGTON US pages 4051 - 4055; GOEDERT, M. ET AL.: 'Cloning and sequencinq of the cDNA encoding a core protein of the paired helical filement of Alzheimer disease : identification as the microtubule associated protein tau' * the whole document * --- | 1,3,4, 13,15-18 | |
| X | JOURNAL OF CELL BIOLOGY vol. 109, no. 4 PI, October 1989, pages 1643 - 1651; HAGESTEDT, T. ET AL.: 'Tau protein becomes long and stiif upon phosphorylation : Correlation between paracrystalline structure and degree of phosphorylation' * page 1650, column 1 * --- | 1,3 | **TECHNICAL FIELDS SEARCHED (Int. Cl.5 )** C07K C12N A61K |
| X | JOURNAL OF BIOCHEMISTRY. vol. 263, no. 16, 5 June 1988, TOKYO JP pages 7703 - 7707; AIZAWA, H. ET AL.: 'Microtubule-binding domain of tau protein' * abstract * --- -/-- | 1,4 | |

The present search report has been drawn up for all claims

| Place of search | Date of completion of the search | Examiner |
|---|---|---|
| THE HAGUE | 06 AUGUST 1992 | CHAMBONNET F.J. |

European Patent
Office

EUROPEAN SEARCH REPORT

Application Number

EP    91 12 0974
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | JOURNAL OF BIOCHEMISTRY. vol. 104, no. 3, 1988, TOKYO JP pages 319 - 321; ISHIGURO, K. ET AL.: 'A novel-tubulin-dependent protein kinase forming a paired helical filament epitope on tau' * the whole document * | 1,5,9,20 | |
| X,D | NEUROSCIENCE LETTERS vol. 128, 22 July 1991, pages 195 - 198; ISHIGURO, K. ET AL.: 'A serine/threonine proline kinase activity is included in the tau protein kinase fraction forming a paired helical filament epitope' * the whole document * | 1,5,9 | |
| T | EMBO JOURNAL. vol. 11, no. 4, April 1992, EYNSHAM, OXFORD GB pages 1593 - 1597; BIERNAT, J. ET AL.: 'The switch of tau protein to an Alzheimer-like state includes the phosphorylation of two serine-proline motifs upstream of the microtubule binding region' * the whole document * | 1-9,13, 20 | TECHNICAL FIELDS SEARCHED (Int. Cl.5) |
| X | JOURNAL OF CELLULAR BIOLOGY vol. 115, no. 3P2, 8 December 1991, BOSTON, USA page 384A; LICHTENBERG, B. ET AL.: 'Alzheimer-type phosphorylation of microtubule- associated protein tau in vitro' * abstract 2230* | 1,5-9, 13, 15-18, 20,21 | |

-/--

The present search report has been drawn up for all claims

| Place of search | Date of completion of the search | Examiner |
|---|---|---|
| THE HAGUE | 06 AUGUST 1992 | CHAMBONNET F.J. |

EPO FORM 1503 03.82 (P0401)

European Patent
Office

**EUROPEAN SEARCH REPORT**

Application Number

EP   91 12 0974
Page 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| T | JOURNAL OF BIOLOGICAL CHEMISTRY. vol. 267, no. 15, 25 May 1992, BALTIMORE US pages 10897 - 10901; ISHIGURO, K. ET AL.: 'Tau Protein Kinase I converts normal tau protein into A68-like component of paired helical filaments' * the whole document * | 5-9,20 | |
| A | WO-A-8 903 993 (MEDICAL RESEARCH COUNCIL) * the whole document * | 1-21 | |
| A | EP-A-0 457 295 (THE ROCKEFELLER UNIVERSITY) * the whole document * | 10,11 | |

TECHNICAL FIELDS SEARCHED (Int. Cl.5)

The present search report has been drawn up for all claims

| Place of search | Date of completion of the search | Examiner |
|---|---|---|
| THE HAGUE | 06 AUGUST 1992 | CHAMBONNET F.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)